# EUROPEAN PATENT SPECIFICATION

(11) **EP 2 331 557 B1**
(45) Date of publication and mention of the grant of the patent: **18.12.2013**
(21) Application number: 09807090.7
(22) Date of filing: 06.08.2009
(51) Int. Cl.: C07H 21/00, C12N 15/00, C12N 7/02

(54) **ALPHAVIRUS PACKAGING CELL LINES**
ALPHAVIRUSVERPACKUNGSZELLINIEN
LIGNÉES CELLULAIRES D' ENCAPSIDATION D'ALPHAVIRUS

(30) Priority: 15.08.2008 US 89399 P
(43) Date of publication of application: 15.06.2011
(73) Proprietor: Novartis AG, 4065 Basel (CH)
(72) Inventor: ZHAI, Weiguo, Emeryville CA 94608-2916 (US); ZUR MEGEDE, Jan, Emeryville CA 94608-2916 (US); BARNETT, Susan, Emeryville CA 94608-2916 (US)
(74) Representative: Marshall, Cameron John
(86) International application number: PCT/US2009/052947
(87) International publication number: WO 2010/019437

(56) References cited:
- US-A1- 2007 166 820
- US-B1- 6 391 632
- US-B1- 6 426 196
- RAYNER ET AL.: 'Alphavirus vectors and vaccination.' REV MED VIROL vol. 12, no. 5, September 2002, pages 279 - 296, XP008035758
- HARDY ET AL.: 'Requirements at the 3' end of the sindbis virus genome for efficient synthesis of minus-strand RNA.' J VIROL vol. 79, no. 8, April 2005, pages 4630 - 4639, XP055005544
- PFEFFER ET AL.: 'The Alphavirus 3'-Nontranslated Region: Size Heterogeneity and Arrangement of Repeated Sequence Elements.' VIROLOGY vol. 240, no. 1, 05 January 1998, pages 100 - 108, XP004446027

### SEQUENCE LISTING

<110> Weiguo Zhai
   Jan zur Megede
   Susan Barnett
<120> ALPHAVIRUS PACKAGING CELL LINES
<130> PAT052653-WO-PCT
<140>
   <141>
<150> US 61/089,399
   <151> 2008-08-15
<160> 15
<170> FastSEQ for Windows Version 4.0
<210> 1
   <211> 20
   <212> PRT
   <213> foot and mouth disease virus
<400> 1
   Gln Leu Leu Asn Phe Asp Leu Leu Lys Leu Ala Gly Asp Val Glu Ser
    1               5                  10                  15
   Asn Pro Gly Pro
               20
<210> 2
   <211> 19
   <212> DNA
   <213> alphavirus
<400> 2
   attttgtttt taatatttc 19
<210> 3
   <211> 17
   <212> DNA
   <213> alphavirus
<400> 3
   ttttgttttt aatattt 17
<210> 4
   <211> 15
   <212> DNA
   <213> alphavirus
<400> 4
   tgtttttaat atttc 15
<210> 5
   <211> 18
   <212> DNA
   <213> alphavirus
<400> 5
   attttatttt taatattt 18
<210> 6
   <211> 18
   <212> DNA
   <213> alphavirus
<400> 6
   attttttttt taatattt 18
<210> 7
   <211> 18
   <212> DNA
   <213> alphavirus
<400> 7
   attttgtttt taaaattt 18
<210> 8
   <211> 18
   <212> DNA
   <213> alphavirus
<400> 8
   attttttttt taatattt 18
<210> 9
   <211> 18
   <212> DNA
   <213> alphavirus
<400> 9
   ttttgttttc aatatttc 18
<210> 10
   <211> 17
   <212> DNA
   <213> alphavirus
<400> 10
   ttttgttttt tatattt 17
<210> 11
   <211> 17
   <212> DNA
   <213> alphavirus
<400> 11
   tttttttttt aatattt 17
<210> 12
   <211> 17
   <212> DNA
   <213> alphavirus
<400> 12
   ttttgttttt attattt 17
<210> 13
   <211> 17
   <212> DNA
   <213> alphavirus
<400> 13
   attttgtttt tattatt 17
<210> 14
   <211> 17
   <212> DNA
   <213> alphavirus
<400> 14
   tttgttttta agatttc 17
<210> 15
   <211> 34
   <212> DNA
   <213> alphavirus
<400> 15
   ttttgtttat aatatttttt tgttttttat attt 34

## Description

### FIELD OF THE INVENTION

The invention relates to the production of recombinant alphavirus particles.

### BACKGROUND OF THE INVENTION

It was previously demonstrated that chimeric alphavirus replicon particles can be generated, wherein the RNA vector is derived from a first alphavirus and the structural "coat" proteins (*e.g*., envelope glycoproteins) are derived from a second alphavirus (see, for example United States patent application serial number 09/236,140, hereinafter the '140 application; see also, U.S. Patents 5,789,245, 5,842,723, 5,789,245, 5,842,723, and 6,015,694; as well as WO 95/07994, WO 97/38087 and WO 99/18226). US 6,391,632 describes isolated nucleic acid molecules comprising an alphavirus nonstructural protein gene which, when operably incorporated into a recombinant alphavirus particle, eukaryotic layered vector initiation system, or RNA vector replicon, has a reduced level of vector-specific RNA synthesis, as compared to wild-type, and the same or greater level of proteins encoded by RNA transcribed from the viral junction region promoter, as compared to a wild-type recombinant alphavirus particle.

Rayner et al. (Rev. Med. Virol., 2002, 12(5):279-96) provides a review of alphavirus vectors and vaccination. However, although previously-described strategies were successful for making several alphavirus chimeras, such chimeric particles are not always produced in commercially viable yields, perhaps due to less efficient interactions between the viral RNA and structural proteins, resulting in decreased productivity

There is a need in the art for packaging cell lines (PCL) that can be used to produce commercial quantities of alphavirus-based replicon particles for vaccine and therapeutic applications.

### SUMMARY OF THE INVENTION

In one aspect, methods of preparing (producing) alphaviral replicon particles as defined by the claims, i.e. using the packaging cell lines of the invention are provided. In certain embodiments, the particles are prepared by introducing any of the replicon and defective helper cassettes described herein into a suitable host cell under conditions that permit formation of the particles. In any of the methods described herein, the defective helper cassettes can include chimeric and/or hybrid structural proteins (or sequences encoding these chimeric/hybrid proteins) as described herein. For example, in certain embodiments, the method comprises introducing into a host cell: (a) an alphavirus replicon RNA derived from one or more alphaviruses, further containing one or more heterologous sequence(s); and (b) at least one separate defective helper RNA(s) encoding structural protein(s) absent from the replicon RNA, wherein at least one of said structural proteins is derived from two or more alphaviruses, wherein alphavirus replicon particles are produced. The replicon RNA can be derived from one or more alphaviruses and the structural proteins can include one or more hybrid proteins, for example, a hybrid capsid protein having an RNA binding domain derived from a first alphavirus and an envelope glycoprotein interaction domain derived from a second alphavirus; and/or a hybrid envelope protein having a cytoplasmic tail portion and a remaining portion, wherein the cytoplasmic tail portion is derived from a first alphavirus and the remaining portion of said envelope glycoprotein derived from one or more alphaviruses different than the first.

Moreover, a method for producing alphavirus replicon particles, comprising introducing into a host cell (a) an alphavirus replicon RNA encoding one or more nonstructural proteins from a first alphavirus, a packaging signal derived from a second alphavirus, (*e.g.*, inserted into a site selected from the group consisting of the junction of nsP3 with nsP4, following the nsP4 open reading frame and a nonstructural protein gene carboxy terminal deletion) and one or more heterologous sequence(s)in nsP3; and (b) at least one separate defective helper RNA(s) encoding structural protein(s) absent from the replicon RNA, wherein at least one of said structural proteins is a capsid protein derived from said second alphavirus, and at least one of said structural proteins is an envelope protein derived from an alphavirus different from said first alphavirus is described.

In yet another aspect, the invention includes alphavirus packaging cell lines as defined by the claims comprising one or more structural protein expression cassettes comprising sequences encoding two or more replicase recognition sites, and one or more structural proteins, wherein at least one of said structural proteins is derived from two or more alphaviruses. One or more structural protein expression cassettes may comprise cDNA copies of a defective helper RNA and, optionally, an alphavirus subgenomic promoter. Further, in any of these embodiments, the defective helper RNA can direct expression of the structural protein(s).

Moreover methods of producing viral replicon particles using packaging cell lines of the invention are described. Typically, the methods comprise introducing, into any of the alphavirus packaging cell lines described herein, any of the alphavirus replicon RNAs described herein, wherein an alphavirus particle comprising one or more heterologous RNA sequence(s) is produced. Thus, in certain embodiments, the RNA will include a packaging signal insertion derived from a different alphavirus. The packaging cell comprises three separate RNA molecules, for example, a first defective helper RNA molecule encodes for viral capsid structural protein(s), a second defective helper RNA molecule encodes for one or more viral envelope structural glycoprotein(s) and a third replicon RNA vector which comprises genes encoding for required nonstructural replicase proteins and a heterologous gene of interest substituted for viral structural proteins, wherein at least one of the RNA molecules includes sequences derived from two or more alphaviruses. Modifications can be made to any one or more of the separate nucleic acid molecules introduced into the cell (e.g., packaging cell) for the purpose of generating chimeric alphavirus replicon particles. For example, a first defective helper RNA can be prepared having a gene that encodes for a hybrid capsid protein as described herein, e.g., the hybrid capsid protein has an RNA binding domain derived from a first alphavirus and a glycoprotein interaction domain from a second alphavirus. A second defective helper RNA may have a gene or genes that encodes for an envelope glycoprotein(s) from a second alphavirus, while the replicon vector RNA is derived from a first alphavirus. In another example, an RNA replicon vector construct is derived from a first alphavirus having a packaging signal from a second alphavirus, inserted for example, in a nonstructural protein gene region that is deleted. The first and second defective helper RNAs have genes that encode for capsid protein or envelope proteins from the second alphavirus. Also, a chimeric alphavirus replicon particle can be made using a first defective helper RNA modified with multiple alphavirus replicase recognition sequences and encoding a capsid protein (derived from a first alphavirus that is the same as the replicon vector source virus) and a second defective helper RNA modified with multiple alphavirus replicase recognition sequences and having a gene that encodes for a hybrid envelope glycoprotein having a cytoplasmic tail fragment from the same alphavirus as the capsid protein of the first helper RNA and a surface-exposed "ectodomain" of the glycoprotein derived from a second alphavirus. The tail fragment interacts with the capsid protein and a chimeric replicon particle having RNA and a capsid derived from a first virus, and an envelope derived primarily from a second virus results.

In another aspect, the invention provides a method for producing alphavirus replicon particles, as defined by the claims, i.e. comprising introducing into a permissible cell, (a) any of the alphavirus replicon RNAs described herein comprising control elements and polypeptide-encoding sequences encoding (i) biologically active alphavirus nonstructural proteins and (ii) a heterologous protein, and (b) one or more defective helper RNA(s) comprising control elements and polypeptide-encoding sequences encoding at least one alphavirus structural protein, wherein the control elements can comprise, in 5' to 3' order, a 5' sequence required for nonstructural protein-mediated amplification, a means for expressing the polypeptide-encoding sequences, and a 3' sequence required for nonstructural protein-mediated amplification, at least two or more alphavirus replication recognition sequences, and a polyadenylate tract,and further wherein one or more of said RNA replicon control elements are different than said defective helper RNA control elements; and incubating said cell under suitable conditions for a time sufficient to permit production of replicon particles. In certain embodiments, the control elements replicon RNA and said defective helper RNA(s) further comprise a subgenomic 5'-NTR. In other embodiments, the subgenomic 5'-NTR of the RNA replicon RNA is different that the subgenomic 5'-NTR of the defective helper RNA; the 5' sequence required for nonstructural protein-mediated amplification of the RNA replicon RNA is different that than the 5' sequence required for nonstructural protein-mediated amplification of the defective helper RNA; the 3' sequence required for nonstructural protein-mediated amplification of the RNA replicon RNA is different that than the 3' sequence required for nonstructural protein-mediated amplification of the defective helper RNA; and/or the means for expressing said polypeptide-encoding sequences of the RNA replicon RNA is different that than the means for expressing said polypeptide-encoding sequences of the defective helper RNA.

Methods for stimulating an immune response within a warm-blooded animal are described comprising the step of administering to a warm-blooded animal a preparation of alphavirus replicon particles according to the present invention expressing one or more antigens derived from at least one pathogenic agent. The antigen may be derived from a tumor cell. The antigen may also be derived from an infectious agent (*e.g*., virus, bacteria, fungus or parasite), such as from a human immunodeficiency virus (HIV) (*e*.*g*. gag, gp120, gp140, gp160 pol, rev, tat, and nef), a hepatitis C virus (HCV) (*e.g.,* C, E1, E2, NS3, NS4 and NS5), an influenza virus (*e.g.,* HA, NA, NP, M), a paramyxovirus such as parainfluenza virus or respiratory syncytial virus or measles virus (*e.g.,* NP, M, F, HN, H), a herpes virus (*e.g*., glycoprotein B, glycoprotein D), a Filovirus such as Marburg or Ebola virus (*e.g*., NP, GP), a bunyavirus such as Hantaan virus or Rift Valley fever virus (*e.g*., G1, G2, N), or a flavivirus such as tick-borne encephalitis virus or West Nile virus (e.g., C, prM, E, NS1, NS3, NS5). Any of the methods described herein can further comprise the step of administering a lymphokine, chemokine and/or cytokine (*e.g*., IL-2, IL-10, IL-12, gamma interferon, GM-CSF, M-CSF, SLC, MIP3α, and MIP3β). The lymphokine, chemokine and/or cytokine can be administered as a polypeptide or can be encoded by a polynucleotide (e.g., on the same or a different replicon that encodes the antigen(s)). Alternatively, a replicon particle of the present invention encoding a lymphokine, chemokine and/or cytokine may be used as a to stimulate an immune response.

Thus, in any of the compositions and methods described herein, sequences are derived from at least two alphaviruses, for example Venezuelan equine encephalitis virus (VEE) and Sindbis virus (SIN).

In addition methods are provided to produce alphavirus replicon particles and reduce the probability of generating replication-competent virus (e.g., wild-type virus) during production of said particles, comprising introducing into a permissible cell an alphavirus replicon RNA and one or more defective helper RNA(s) encoding at least one alphavirus structural protein, and incubating said cell under suitable conditions for a time sufficient to permit production of replicon particles, wherein said replicon RNA comprises a 5' sequence required for nonstructural protein-mediated amplification, sequences which, when expressed, code for biologically active alphavirus nonstructural proteins, a means to express one or more heterologous sequences, a heterologous sequence that is a protein-encoding gene, said gene being the 3' proximal gene within the replicon, a 3' sequence required for nonstructural protein-mediated amplification, multiple alphavirus replicase recognition sequences, a polyadenylate tract, and optionally a subgenomic 5'-NTR; and wherein said defective helper RNA comprises a 5' sequence required for nonstructural protein-mediated amplification, a means to express one or more alphavirus structural proteins, a gene encoding an alphavirus structural protein, said gene being the 3' proximal gene within the defective helper, a 3' sequence required for nonstructural protein-mediated amplification, a polyadenylate tract, and optionally a subgenomic 5'-NTR; and wherein said replicon RNA differs from at least one defective helper RNA in at least one element selected from the group consisting of a 5' sequence required for nonstructural protein-mediated amplification, a means for expressing a 3' proximal gene, a subgenomic 5' NTR, and a 3' sequence required for nonstructural protein-mediated amplification.

These and other aspects and embodiments of the invention will become evident upon reference to the following detailed description, attached figures and various references set forth herein that describe in more detail certain procedures or compositions (e.g., plasmids, sequences, etc.).

### BRIEF DESCRIPTION OF THE FIGURES

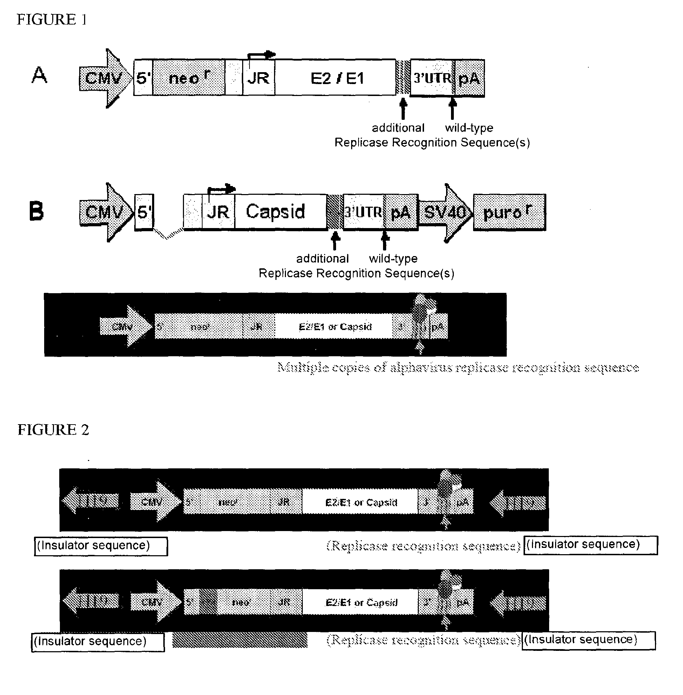

**FIG. 1****.** A schematic depiction of an embodiment of defective helper cassettes of the invention for expression of glycoprotein (**FIG. 1A**) or capsid protein (**FIG. 1B**). In this embodiment, four copies of alphavirus replicase recognition sequence are inserted after the stop codon of an alphaviral structural protein sequence and upstream of the 3'UTR sequence, as indicated by the arrow. The fifth alphavirus replicase recognition sequence is located at the 3'end and contained within the wild-type 3'UTR sequence. CMV, cytomegalovirus promoter; neo^{r}, neomycin resistance gene; JR, alphavirus subgenomic promoter, SV40, simian virus promoter.

**FIG. 2**. A schematic depiction of a structural protein expression cassette of the invention. Insulator sequences are labeled as "H19," and the Ex-2 intron sequence is labeled as "Ex-2." The inclusion of these elements are demonstrated as examples for the E2/E1 glycoprotein expression cassette and can be implemented in the same fashion for the capsid structural protein expression cassette as depicted in **FIG. 1B** and **FIG. 6****.**

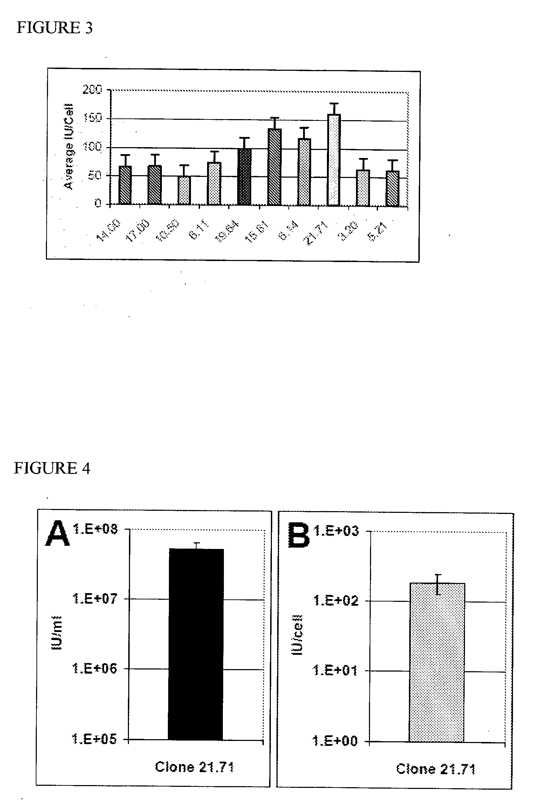

**FIG. 3****.** Graph showing productivity in infectious units (IU) per cell of several representative packaging cell line clones. The error bars represent variations of multiple independent assays in productivity of the clones represented.

**FIGS. 4A-B****.** Graphs showing expression levels of a packaging cell clone 21.71 measured in infectious units per ml (**FIG. 4A**) and infectious units per cell (**FIG. 4B**). The standard error was calculated from four independent experiments.

**FIG. 5****.** Photomicrographs of intact virion-sized (60-70nm) as well as smaller core-sized particles produced in PCL clone 21.71. Bar=100nm.

**FIG. 6****.** A schematic depiction of a structural protein expression cassette of the invention comprising selectable marker gene (puromycin resistance) and a coding sequence for the foot-and-mouth disease virus (FMDV) 2A protease 19 amino acid sequence inserted between the alphaviral 5' end sequence and the puromycin resistance gene.

### DETAILED DESCRIPTION OF THE INVENTION

This invention provides alphavirus packaging cell lines (PCL) useful for commercial production of recombinant alphavirus particles. An alphavirus packaging cell contains one or more alphavirus structural protein expression cassettes and produces recombinant alphavirus particles after introduction of a vector (*e.g*., an alphavirus RNA vector replicon, eukaryotic layered vector initiation system, or recombinant alphavirus particle) as described below.

The invention also provides structural protein expression cassettes which can be used to produce the alphavirus PCL. Structural protein expression cassettes of the invention encode either the alphavirus capsid protein (C) or the alphavirus glycoprotein (E2/E1); thus, two separate structural protein expression cassettes are used in a PCL to produce alphavirus-based replicon particles. Use of such "split helper" constructs reduces the opportunity for recombination with vector RNA and the subsequent generation of contaminating wild-type virus.

### Structural protein expression cassettes

A structural protein expression cassette according to the invention encodes an alphavirus structural protein - either capsid (C) or glycoprotein (E2/E1), but not both - and comprises at least two, preferably five copies of an alphavirus replicase recognition sequence. Structural protein expression cassettes typically comprise, from 5' to 3', a 5' sequence which initiates transcription of alphavirus RNA, an alphavirus subgenomic region promoter, a nucleotide sequence encoding the alphavirus structural protein, a 3' untranslated region, and a polyA tract. A 3'CSE, conserved sequence element of 19NT is located at the 3'end of the 3'UTR. The presence of the 3'CSE is essential for alphavirus minus-strand RNA replication and functions as promoter element for the replicase complex. Increasing the number of 3'CSE sequences at the 3'end of the structural protein expression cassette can improve the recognition of the promoter region by the replicase complex which is provided in *trans,* which results in increased expression of structural proteins and, therefore increased productivity of a packaging cell line containing the expression cassettes. In the wild-type virus, the first replicase recognition sequence is located adjacent to the polyA tract. In expression cassettes of the invention, four additional copies of alphavirus replicase recognition sequence are inserted after the stop codon of the capsid or E2/E1 alphavirus structural protein coding sequence and just upstream of the 3'UTR sequence. In some embodiments, the 3'UTR comprises 108 bp of the VEE Trinidad Donkey strain 3'UTR. Typically the fifth alphavirus replicase recognition sequence is located at the 3'end adjacent to the polyA segment and contained within the wild-type 3'UTR sequence. In some embodiments, one skilled in the art can create structural protein expression cassettes to comprise less than five or more than five replicase recognition sequences (3'CSE). The additional replicase recognition sequences can be located either upstream of the wild-type or of a shorter 3'UTR or at any point between the coding sequence for the structural protein and the first replicase recognition sequence.

Any alphavirus replicase recognition sequence can be used including, but not limited to, those obtained from Sindbis virus, Venezuelan equine encephalitis virus, Semliki Forest virus, Ross River virus, and Western equine encephalitis virus. Examples of such replicase recognition sequences are shown in SEQ ID NOS:2-15. Replicase recognition sequences which are at least 78%, 85%, 89%, or 94% identical to these sequences also can be used in structural protein expression cassettes of the invention. Replicase recognition sequences included in structural protein expression cassettes of the invention need not be identical, and any combination of replicase recognition sequences can be used.

It is useful to include in a structural protein expression cassette a selectable marker, such as an antibiotic resistance gene (*e.g*., neomycin, puromycin, or hygromycin resistance). Preferably two structural protein expression cassettes encoding the capsid and glycoprotein, respectively, comprise two different selectable markers. In some embodiments, a glycoprotein expression cassette comprises a neomycin resistance gene, and a capsid expression cassette has a puromycin resistance gene. In other embodiments expression cassettes comprise an FMDV2a/Puro sequence.

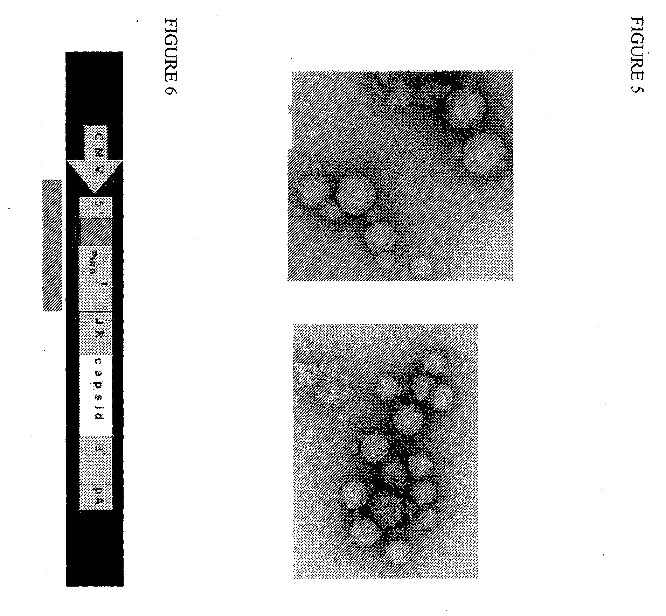

Some structural protein expression cassettes comprise a nucleotide sequence encoding the foot-and-mouth disease virus (FMDV) 2A protease (QLLNFDLLKLAGDVESNPGP; SEQ ID NO:1), which has co-translational cleavage activity, between the 5' sequence which initiates transcription of alphavirus RNA and the alphavirus subgenomic region promoter. See **FIG. 6**.

In some embodiments an intron sequence (*e.g*., Ex-2) is inserted immediately after the alphavirus 5' sequence. **FIG. 2**.

Structural protein expression cassettes can be assembled from known elements using standard recombinant DNA techniques. *See, e.g.*, U.S. Patents 5,843,723; 6,329,201; and 6,426,196.

### Production of packaging cells

Packaging cells of the invention comprise at least one structural protein expression cassette of the invention. Preferably a packaging cell comprises two structural protein expression cassettes encoding the capsid and the glycoprotein, respectively. The packaging cell may be of mammalian or non-mammalian origin (*e.g*., insect). Within preferred embodiments, the packaging cell is stably transformed with the structural protein expression cassette(s). PerC6 cells are useful for producing packaging cells. PerC6 (PER.C6) is a cell line developed by Crucell N.V. It is an immortalized cell line derived from a single human retina-derived cell, and its history is well documented. Other useful cells include, but are not limited to, Probiogen avian cells (Vero, Vivalis duck cells), 293 HEK, and insect cells (*e.g*., SF9).

Structural protein expression cassettes can be introduced into cells using standard recombinant DNA techniques, including transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun" methods, and DEAE- or calcium phosphate-mediated transfection. Structural protein expression cassettes typically are introduced into a host cell as DNA molecules, but can also be introduced as *in vitro*-transcribed RNA. Each expression cassette can be introduced into cells separately or substantially simultaneously.

### Methods of producing recombinant alphavirus particles

The invention also provides methods of producing recombinant alphavirus particles. A recombinant alphavirus particle is a virion unit containing an alphavirus RNA vector replicon. An alphavirus RNA vector replicon is an RNA molecule which can direct its own amplification *in vivo* in a target cell. The replicon encode the polymerase(s) necessary to catalyze RNA amplification (nsP1, nsP2, nsP3, nsP4) and contains cis RNA sequences required for replication which are recognized and utilized by the encoded polymerase(s). An alphavirus RNA vector replicon typically contains the following ordered elements: 5' viral sequences required in cis for replication, sequences which encode biologically active alphavirus nonstructural proteins (nsP1, nsP2, nsP3, nsP4), 3' viral sequences required in cis for replication, and a polyadenylate tract. The alphavirus RNA vector replicon also may contain a viral subgenomic "junction region" promoter, which may, in certain embodiments, be modified in order to increase or reduce viral transcription of the subgenomic fragment, and heterologous sequence(s) to be expressed.

Generally, the recombinant alphavirus particle comprises one or more alphavirus structural proteins, a lipid envelope and an RNA vector replicon. Preferably, the recombinant alphavirus particle contains a nucleocapsid structure that is contained within a host cell-derived lipid bilayer, such as a plasma membrane, in which one or more alphaviral envelope glycoproteins are embedded. The particle may also contain other components (e.g., targeting elements such as biotin, other viral structural proteins, hybrid envelopes, or other receptor binding ligands) which direct the tropism of the particle from which the alphavirus was derived (see Gardner et al., J. Virol. 74, 11849-57, 2000).

Recombinant alphavirus particles are produced by introducing a vector into a packaging cell line as described above. The vector can be, *e.g.*, an alphavirus RNA vector replicon, eukaryotic layered vector initiation system, or a recombinant alphavirus particle; see U.S. Patents 6,329,201 and 6,015,686.

The vector typically encodes a heterologous protein of interest. A wide variety of heterologous proteins may be encoded in the vector, including for example palliatives such as lymphokines, toxins, prodrugs, antigens which stimulate an immune response, ribozymes, and proteins which assist or inhibit an immune response, as well as antisense sequences (or sense sequences for antisense applications). In some embodiments of the invention the vectors provided herein may contain (and express, in some embodiments) two or more heterologous sequences.

The vector encoding the heterologous protein typically comprises a 5' sequence which initiates transcription of an alphavirus RNA, sequences which encode biologically active alphavirus nonstructural proteins (nsP1, nsP2, nsP3, nsP4), an alphavirus RNA polymerase recognition sequence, a heterologous sequence to be expressed, and a polyA *tract. See, e.g.,* the alphavirus vector constructs, RNA vector replicons, and eukaryotic layered vector initiation systems described in U.S. Patent 6,329,201.

The chimeric alphavirus particles generated by the packaging cells of the invention can be used to deliver a wide variety of nucleotide sequences including, for example, sequences which encode lymphokines or cytokines (*e*.*g*., IL-2, IL-12, GM-CSF), prodrug converting enzymes (*e*.*g*., HSV-TK, VZV-TK), antigens which stimulate an immune response (*e*.*g*., HIV, HCV, tumor antigens), therapeutic molecules such as growth or regulatory factors (*e*.*g*., VEGF, FGF, PDGF, BMP), proteins which assist or inhibit an immune response, as well as ribozymes and antisense sequences. The above nucleotide sequences include those referenced previously (*e*.*g*., U.S. 6,015,686, WO 9738087 and WO 9918226), and may be obtained from repositories, readily cloned from cellular or other RNA using published sequences, or synthesized, for example, on an Applied Biosystems Inc. DNA synthesizer (*e*.*g*., APB DNA synthesizer model 392 (Foster City, CA)).

For purposes of the present invention, virtually any polypeptide or polynucleotide can be used. Antigens can be derived from any of several known viruses, bacteria, parasites and fungi, as well as any of the various tumor antigens or any other antigen to which an immune response is desired. Furthermore, for purposes of the present invention, an "antigen" refers to a protein that includes modifications, such as deletions, additions and substitutions (generally conservative in nature), to the native sequence, so long as the protein maintains the ability to elicit an immunological response. These modifications may be deliberate, as through site-directed mutagenesis, or may be accidental, such as through mutations of hosts that produce the antigens.

Antigens may be used alone or in any combination. (See, *e*.*g*., WO 02/00249 describing the use of combinations of bacterial antigens). The combinations may include multiple antigens from the same pathogen, multiple antigens from different pathogens or multiple antigens from the same and from different pathogens. Thus, bacterial, viral, tumor and/or other antigens may be included in the same composition or may be administered to the same subject separately. It is generally preferred that combinations of antigens be used to raise an immune response.

Non-limiting examples of bacterial pathogens include diphtheria (*See, e.g.,* Chapter 3 of Vaccines, 1998, eds. Plotkin & Mortimer (ISBN 0-7216-1946-0), staphylococcus (*e.g., Staphylococcus aureus* as described in Kuroda et al. (2001) Lancet 357:1225-1240), cholera, tuberculosis, *C*. *tetani,* also known as tetanus (*See, e.g.,* Chapter 4 of Vaccines, 1998, eds. Plotkin & Mortimer (ISBN 0-7216-1946-0), Group A and Group B streptococcus (including *Streptococcus pneumoniae, Streptococcus agalactiae* and *Streptococcus pyogenes* as described, for example, in Watson et al. (2000) Pediatr. Infect. Dis. J. 19:331-332; Rubin et al. (2000) Pediatr Clin. North Am. 47:269-284; Jedrzejas et al. (2001) Microbiol Mol Biol Rev 65:187-207; Schuchat (1999) Lancet 353:51-56; GB patent applications 0026333.5; 0028727.6; 015640.7; Dale et al. (1999) Infect Dis Clin North Am 13:227-1243; Ferretti et al. (2001) *PNAS* USA 98:4658-4663), pertussis *(See, e.g.,* Gusttafsson et al. (1996) N. Engl. J. Med. 334:349-355; Rappuoli et al. (1991) TIBTECH 9:232-238)*,* meningitis, *Moraxella catarrhalis (See, e.g.,* McMichael (2000) Vaccine 19 Suppl. 1:S101-107) and other pathogenic states, including, without limitation, Neisseria meningitides (A, B, C, Y), *Neisseria gonorrhoeae* (*See, e.g.,* WO 99/24578; WO 99/36544; and WO 99/57280), Helicobacter pylori *(e.g.,* CagA, VacA, NAP, HopX, HopY and/or urease as described, for example, WO 93/18150; WO 99/53310; WO 98/04702) and *Haemophilus* /*influenza.* Hemophilus influenza type B (HIB) (See, e.g., Costantino et al. (1999) Vaccine 17:1251-1263), *Porphyromonas gingivalis* (Ross et al. (2001) Vaccine 19:4135-4132) and combinations thereof.

Non-limiting examples of viral pathogens include meningitis, rhinovirus, influenza (Kawaoka et al., Virology (1990) 179:759-767; Webster et al., "Antigenic variation among type A influenza viruses," p. 127-168. In: P. Palese and D.W. Kingsbury (ed.), Genetics of influenza viruses. Springer-Verlag, New York), respiratory syncytial virus (RSV), parainfluenza virus (PIV), and the like. Antigens derived from other viruses will also find use in the present invention, such as without limitation, proteins from members of the families Picomaviridae (e.g., polioviruses, etc. as described, for example, in Sutter et al. (2000) Pediatr Clin North Am 47:287-308; Zimmerman & Spann (1999) Am Fam Physician 59:113-118; 125-126); Caliciviridae; Togaviridae (e.g., rubella virus, dengue virus, etc.); the family Flaviviridae, including the genera flavivirus (*e*.*g*., yellow fever virus, Japanese encephalitis virus, serotypes of Dengue virus, tick borne encephalitis virus, West Nile virus); pestivirus (*e*.*g*., classical porcine fever virus, bovine viral diarrhea virus, border disease virus); and hepacivirus (*e*.*g*., hepatitis A, B and C as described, for example, in U.S. Patent Nos. 4,702,909; 5,011,915; 5,698,390; 6,027,729; and 6,297,048); Parvovirsus (*e*.*g*., parvovirus B19); Coronaviridae; Reoviridae; Bimaviridae; Rhabodoviridae (e.g., rabies virus, etc. as described for example in Dressen et al. (1997) Vaccine 15 Suppl:s2-6; MMWR Morb Mortal Wkly Rep. 1998 Jan 16:47(1):12, 19); Filoviridae; Paramyxoviridae (e.g., mumps virus, measles virus, rubella, respiratory syncytial virus, etc. as described in Chapters 9 to 11 of Vaccines, 1998, eds. Plotkin & Mortimer (ISBN 0-7216-1946-0); Orthomyxoviridae (e.g., influenza virus types A, B and C, etc. as described in Chapter 19 of Vaccines, 1998, eds. Plotkin & Mortimer (ISBN 0-7216-1946-0),.); Bunyaviridae; Arenaviridae; Retroviradae (e.g., HTLV-1; HTLV-11; HIV-1 (also known as HTLV-III, LAV, ARV, HTI,R, etc.)), including but not limited to antigens from the isolates HIVI11b, HIVSF2, HIVLAV, HIVI-AL, I-IIVMN); HIV- I CM235, HIV- I IJS4; HIV-2; simian immunodeficiency virus (SIV) among others. Additionally, antigens may also be derived from human papilloma virus (HPV) and the tick-borne encephalitis viruses. See, e.g. Virology, 3rd Edition (W.K. Joklik ed. 1988); Fundamental Virology, 2nd Edition (B.N. Fields and D.M. Knipe, eds, 1991), for a description of these and other viruses.

Antigens from the hepatitis family of viruses, including hepatitis A virus (HAV) *(See, e.g.,* Bell et al. (2000) Pediatr Infect Dis. J. 19:1187-1188; Iwarson (1995) APMIS 103:321-326), hepatitis B virus (HBV) *(See, e.g.,* Gerlich et al. (1990) Vaccine 8 Suppl:S63-68 & 79-80), hepatitis C virus (HCV), the delta hepatitis virus (HDV), hepatitis E virus (HEV) and hepatitis G virus (HGV), can also be conveniently used in the techniques described herein. By way of example, the viral genomic sequence of HCV is known, as are methods for obtaining the sequence. See, e.g., International Publication Nos. WO 89/04669; WO 90/11089; and WO 90/14436. Also included in the invention are molecular variants of such polypeptides, for example as described in PCT/US99/31245; PCT/US99/31273 and PCT/US99/31272.

Non-limiting examples of tumor antigens include antigens recognized by CD8+ lymphocytes (*e*.*g*., melanoma-melanocyte differentiation antigens such as MART-1, gp100, tyrosinase, tyrosinase related protein-1, tyrosinase related protein-2, melanocyte-stimulating hormone receptor; mutated antigens such as beta-catenin, MUM-1, CDK-4, caspase-8, KIA 0205, HLA-A2-R1701; cancer-testes antigens such as MAGE-1, MAGE-2, MAGE-3, MAGE-12, BAGE, GAGE and NY-ESO-1; and non-mutated shared antigens over expressed on cancer such as alpha-fetoprotein, telomerase catalytic protein, G-250, MUC-1, carcinoembryonic antigen, p53, Her-2-neu) as well as antigens recognized by CD4+ lymphocytes (*e*.*g*., gp100, MAGE-1, MAGE-3, tyrosinase, NY-ESO-1, triosephosphate isomerase, CDC-27, and LDLR-FUT). See, also, WO 91/02062, U.S. Patent No. 6,015,567, WO 01/08636, WO 96/30514, U.S. Patent No. 5,846,538 and U.S. Patent No. 5,869,445.

In certain embodiments, the tumor antigen(s) may be used. Tumor antigens are derived from mutated or altered cellular components. After alteration, the cellular components no longer perform their regulatory functions, and hence the cell may experience uncontrolled growth. Representative examples of altered cellular components include ras, p53, Rb, altered protein encoded by the Wilms' tumor gene, ubiquitin, mucin, protein encoded by the DCC, APC, and MCC genes, as well as receptors or receptor-like structures such as neu, thyroid hormone receptor, platelet derived growth factor (PDGF) receptor, insulin receptor, epidermal growth factor (EGF) receptor, and the colony stimulating factor (CSF) receptor. These as well as other cellular components are described for example in U.S. Patent No. 5,693,522 and references cited therein.

### EXAMPLE 1

### GLP Grade DNA preparation

Bacteria (strain MM294-1; CMCC#1872) are first streaked on Veggie Agar Plate [10g Veggie peptone, 5g Veggie yeast extract, 10g NaCl, 20g Agar per liter]. Single colonies are then expanded in Veggie LB [10g Veggie peptone, 5g Veggie yeast extract, 10g NaCl per liter] and used to make competent cells in 50mM CaCl₂ (Sigma C5670). Plasmid DNA to be used in PCL derivation are first diluted 1:200 with water (Hyclone Hypure WFI, cat#SH30221.10) and then transformed into MM294-1 competent cells. Single colonies picked from Veggie LB agar/Ampicillin or Kanamycin plates are expanded into 100ml Veggie LB/Ampicillin or Kanamycin culture, then single colonies are streaked on Veggie LB agar/Ampicillin or Kanamycin plate. This process is carried out five times, after which manufacturing cell lines are generated and ready to be stored and grown for large scale DNA isolation (1-3L culture). Large scale plasmid preparation is performed using a Qiagen kit, and final DNA was resuspended in WIF water. Plasmid concentration is determined using a spectrophotometer.

### EXAMPLE 2

### PerC6 Alpha PCL Derivation

### Transfection

PerC6 cells are obtained from Crucell N.V. and are plated at 2.5e6/10 cm dish and grown overnight in DMEM supplemented with 10% FBS and 10mM MgCl₂. Sixty-three microliters of Lipofectamine 2000CD (Invitrogen) are diluted dropwise into 0.5 ml of unsupplemented DMEM and incubated at room temperature for five minutes. Twenty-four micrograms of DNA are diluted into 0.5 ml unsupplemented DMEM and incubated at room temperature for five minutes. After the five minute incubation, the Lipofectamine mixture is added to the diluted DNA and incubated for another 20 minutes at room temperature. During this time, the growth media is removed from the PerC6 10 cm dish and replaced with unsupplemented DMEM. After the 20 minute incubation, the DNA/Lipofectamine mixture is added dropwise to the 10 cm dish and allowed to incubate overnight at 37°C, 10% CO₂.

The next day, the media containing the transfection reagent is removed from the 10 cm dish and replaced with growth media. On day 4, the contents of the 10 cm dish are split into new 10 cm dishes at a ratio of 1:10-1:40, depending on cell density and viability of transfection plate. Selective media (1000 µg/ml G418) was added, then the dishes are incubated for 10-20 days until distinct colonies appeared.

### Selection of Clones and Propagation

Once distinct colonies formed on the 10 cm dishes, the medium is removed from the 10 cm dish and replaced with 20 mls fresh selective media. The colonies are picked using a 200 µl pipette using a microscope. The colonies are transferred to a 96 well plate in selective media. At least 2,000-5,000 colonies typically are picked per construct, and plates are typically incubated at 37°C and 10% CO₂ for 10-15 days, or until the majority of the wells on the plate are almost confluent. If necessary, the fastest growing clones within the 96 well plate can be split, so that the slower growing clones have a chance to catch up.

### Primary Screening

ELISA and immunoflourescence assays are used for primary screening of the clones using rabbit polyclonal and mouse monoclonal antibodies against Sindbis capsid, Sindbis glycoprotein E1, and whole Sindbis virus. Replica 96 well plates are generated for the assays. Selection criteria for the ELISA is based on OD/clone, and the top 20% of the clones of each 96 well plate selected. IFA data is based on viewing of the GFP expression/well. The top 20% of clones are selected based on these results. A high majority of the clones identified by IFA were also identified by ELISA, the ELISA identified more clones than the IFA due to higher sensitivity.

### Secondary Screening

Once the top PerC6 Gly clones are identified by a combination of IFA and ELISA results, secondary screening is carried out by titer assay using BHKV cells. All clones are screened at least twice by titer assay before the top 15 clones are identified.

### Subcloning

Once the top 15 Gly Perc6 clones are identified, they are subcloned by limiting dilution cloning into 96 well plates containing selective media. A total of approximately 3000 subclones are screened using ELISA and titer assay results to determine the two best PerC6.Gly subclones (NC3.44 and 12 E8.44).

### Secondary Transfection

Both PerC6 Gly subclones NC3.44 and 12E8.44 are re-transfected with the M19 DH Cap construct (**FIG. 1B**) to establish a stable cell line containing both the Gly and Cap genes. PerC6 Gly subclone 12E8.44 is also re-transfected with the M19 DH FMDV Cap construct (**FIG. 6**) for comparison with the M19 DH Cap construct. As the Gly clones are already under G418 selection, a puromycin killing curve is carried out on subclone NC3.44 to determine the level of puromycin needed to be added to the current selection media. For secondary transfections the selection media contains 800-1000 µg/ml G418 and 0.25-0.5 µg ml puromycin.

Gly parent clones are re-transfected according to the same protocol as the primary PerC6 transfections.

### PerC6 Glycoprotein Clone IFA Straining

PerC6 Clones in 96well are infected with alphavirus GFP VEE/SIN particles at a MOI of 10 and then incubated at 37⁻C for 20-24 hr. Medium from these 96well plates are then removed and cells are washed once with 1xPBS. Cells were then fixed by adding 100% methanol at room temperature. Plates were quickly moved to -200°C freezer and kept there overnight.

The next morning, the methanol is removed and the plates are air dried for about 10-20 min. Plates are then rinsed once with 1xPBS once. Diluted first antibody (rabbit polyclonal anti-E1_2wp3, 1:500 dilution in 1xPBS with 2% BSA, 80 µl/well) is added, and the plates are incubated at room temperature for 2 hr. At the end of incubation, the plates are washed three times with a 5 minute incubation with 1xPBS at room temperature. Diluted second antibody (1:2,500 dilution in 1xPBS with 2% BSA, 80 µl/well; for Red Dye, Alexa Fluor 568 goat anti-rabbit IgG is used [Invitrogen A11036]) is added, and the plates are incubated in the dark at room temperature for 2 hr. At the end of incubation, the plates are washed three times with a 5 minute incubation with 1xPBS at room temperature.

### EXAMPLE 3

### Titer assay for secondary screening of PerC6 Gly clone

### Infection

Approximately 6e4 cells are plated in each well of a 96-well plate and allowed to attach for a minimum of 24 hrs at 37°C, 10% CO₂. Cells are then induced with MOI=10, using the highest cell counts as the basis for calculation (virus stocks were diluted in PerC6 medium with 1% FBS, 50 µl/well) and incubated at 37°C 2-2.5hrs. At the end of incubation, 150 µl of complete PerC6 medium with 10% FBS is added, then incubation continued for 16-24 hrs at 37°C, 10% CO₂. For plates to be used in capture ELISA, medium is removed, then 165 µl of lysis buffer [25mM Tris HCl, 1% Triton X-100, complete protease inhibitor used at 2 tablets/100ml] is added. After 30 minutes at room temperature, the plates are stored at -800°C.

### Transfection

PerC6 clones are plated in 12well plates the day before transfection. Transfection is done with LT1 (Mirus Bio Corp). Six microliters of LT1 was diluted in 100 µl OptiMEM and incubated at room temperature for 15-30 min. After incubation, 1µg of DH-cap (VCP-dlnsps-Puro-Scap) and 1µg replicon DNA (VCP-delXhoGFP) are added. The mixture is mixed gently and then incubated at room temperature for 30 minutes. One milliliter of complete PerC6 medium is then added to the transfection mix. The old medium is removed from the 12 well plates and replaced by the diluted transfection mixture.

### Titering

Supernatant (about 0.5 ml) is harvested after about 36-40hrs (essentially in the morning on day three), spun quickly, and added (100 µl/well) to BHK cells plated at 4e5/well in 12 well plates for 1-1.5hrs, with intermittent gentle mixing. After infection, the infection medium is removed, the cells are washed once with 1ml PBS, then 1ml of BHKV complete medium with 10% FBS is added to each well. After 20-24 hr, FACS is performed, then the viral titer is calculated.

### EXAMPLE 4

### Capture ELISA assays for capsid and glycoproteins

ELISA plates are coated with 8920 polyclonal rabbit anti-Sindbis sera (1:10,000 in 1xPBS) and incubated at 4°C for at least 14 hours. Plates are then washed three times with 200µl/well of 1x wash buffer, patted dry, and incubated with 150 µl blocking buffer (0.3% Tween 20 and 2% goat serum in PBS) at 37°C for 1 hour. Dilutions of baculovirus-produced capsid standards are made in PBS (500ng/ml, 50ng/ml, 5ng/ml, and 0.5ng/ml). Dilutions of 293 cell-produced E1 standards also are made in PBS (1 µg/ml, 0.25 µg/ml, 0.06 µg/ml, and 0.017 µg/ml). After blocking, 100 µl/well of sample or standard are added and the plates are incubated overnight at 4 °C or at RT for 1.5 hours.

Plates are washed with 1xPBS. Mouse capsid and E1 mAb are diluted 1:10,000 with blocking buffer, and 100 µl are added to each well. Wells are incubated at RT for 1 hour. A 1:130,000 dilution of goat anti-mouse peroxidase conjugate is prepared in blocking buffer. Plates are washed 3 times with 1xPBS, then 100 µl of diluted anti-mouse HRP are added to each well of all plates. Plates are incubated for 1hr at RT and washed three times with PBS. Equal parts of TMB peroxidase substrate and TMB peroxidase solution are mixed and added to each well of all plates (100 µl/well). Plates incubate at room temperature for 15 minutes. Positive wells turn blue. Phosphoric acid (3M, 100 µl/well) is added to stop the reaction, which turns the blue to yellow. The plates are read on a Bio-Tek PowerWave x340 reader at 450 nm and 600 nm using KC4 software. Background calculation is based on geomean of negative samples plus 3 standard deviation. An endpoint read-out OD value above this value is considered to be positive.

### EXAMPLE 5

### Sucrose gradient purification of replicon particles

Stable PCL clones are induced with replicon particles at 2 MOI. The virus-containing cell culture supernatant is collected after 48 h incubation and cleared of cell debris by a 15 min centrifugation in a Beckman JA-10 rotor at 4°C for 30 min at 5,000rpm. The virus particles in the cleared supernatant are then sedimented through a 10% and 20% (w/v) sucrose step gradient (dissolved in TNE buffer) in a Beckman SW 28 rotor at 25,000 r.p.m. for 1.5 h at 4 °C. The virus pellets are then resuspended in TNE buffer (50 mM Tris HCl, pH 7±4, 100 mM NaCl, 0±1 mM EDTA) containing 1% glycerol. The resuspended virus pellet is immediately prepared for scanning transmission electron microscopy (STEM).

### EXAMPLE 6

### Immunoblotting of purified replicon particles

Cells are collected by trypsinization and pelleted. The pellet is lysed with 100 µl lysis buffer on ice for 10 min and centrifuged at 14,000 rpm (16,000 g) in an Eppendorf microfuge for 10 min at 4°C. The supernatant is transferred to a new tube, and 20 µl of the cleared lysate is boiled and run on a SDS-PAGE at 10-15 V (constant voltage) for 60 min. The protein is then transferred on a nitrocellulose membrane using the semi dry method. The transferred membrane is then immersed in blocking buffer (1:1 Oddesey blocking buffer in PBS-Tween; LI-COR Biosciences, Lincoln, NE) overnight at 4°C. The primary antibody (rabbit anti-E1 and rabbit anti-Cap at 1:1000; Novartis) is diluted in blocking buffer for 60 min at room temp. The membrane is then washed 3 x 10 min with 0.05% Tween 20 in PBS, incubated with the secondary antibody (goat anti rabbit IRDye-680; LI-COR) diluted in blocking buffer for 45 min at room temp, and washed 3 x 10 min with 0.05% Tween 20 in PBS. Detection is performed with the Odyssey Infrared Imaging System (LI-COR).

Clean GLP-grade DNA is prepared as described in Example 1 and transfected into PerC6 cells as described in Example 2. Approximately 4,000 clones are selected and propagated from the second transfection. These clones are analyzed first with capture ELISA using rabbit polyclonal antibody against Sindbis capsid protein. The top 20% of clones with high capsid and E1 expression as determined by capture ELISA were further analyzed by titer assay.

As determined by the titer assay on BHKV cells, we found that the top PerC6 PCL clones are able to produce >100 IU/cell of alphavirus GFP particles within 22-46 hours post infection (MOI=0.5-2). Tissue culture supernatant collected from the top clones 22-46 hrs post infection typically contains 10e7∼110e8 infectious particles per ml.

### EXAMPLE 7

### Post-,screening characterization of top clones

After the primary and secondary high throughput screening procedures to select the clones with highest expression and productivity, a more detailed characterization is performed for the top clones, in particular clone 21.71. The productivity levels determined in previous screens were confirmed. In four independent experiments 21.71 cells are induced with 1-2 MOI and supernatants are harvested 48h later and tested on BHK cells to determine productivity per ml supernatant (average 5.17e7 IU/ml) and per cell (average 125 IU/cell).

The expression levels of Capsid and Glycoprotein are shown in Table 1. Cell lysates of clones induced with 1 MOI are harvested 48 post induction and evaluated by capture ELISA. Clone 21.71 demonstrated an equimolar ratio of Capsid and Glycoprotein, comparable to the ratio found in wild-type virus. A different clone (15.61) with a higher Glycoprotein expression level was included to demonstrate the varying Cap/Gly ratios in these clones.

**Table 1: Cap and Gly expression of stable PCL clones**

| Sample | Cap (µg/ml) | Glyco (µg/ml) | Glyco/Cap |
|---|---|---|---|
| Clone 21.71 | 1.04 | 0.99 | 0.95 |
| Clone 15.71 | 0.95 | 2.68 | 2.83 |
| Naïve Perc6 | 0.00 | 0.00 | NA |

The qualitative characterization of the replicon particles produced by stable clones is performed by immunoblots of purified particle preparations. Supernatants of induced cells are harvested 48h post induction and concentrated by a sucrose step gradient. Capsid and Glycoprotein from purified particles produced in the stable clone 21.71 have the same pattern and size as particle produced by a transient transfection of a pCMVKm2.CapGly fusion cassette as well as replicon particles produced in BHK cells using triple RNA transfection . Uninduced cells of clone 21.71 did not produce detectable amounts of particles.

The morphology of particles produced from the top PCL clones is evaluated by electron microscopy. Particles are concentrated through sucrose gradient sedimentation and determined by STEM (scanning transmission electron microscopy) to be structurally similar to alphavirus particles. **FIG. 5**.

## Claims

1. An alphavirus structural protein expression cassette, comprising, from 5' to 3':
a 5' sequence which initiates transcription of alphavirus RNA;
an alphavirus subgenomic region promoter;
a nucleotide sequence encoding either an alphavirus envelope polypeptide or an alphavirus capsid polypeptide;
a 3'untranslated region; and
a polyA tract,
wherein the alphavirus structural protein expression cassette comprises a first replicase recognition sequence adjacent to the polyA tract and a second replicase recognition sequence located between the first replicase recognition sequence and the nucleotide sequence.

2. The alphavirus structural protein expression cassette of claim 1 wherein the first and second replicase recognition sequences are at least 78% identical to a nucleotide sequence selected from the group consisting of SEQ ID NOS:2-15,
are at least 84% identical to a nucleotide sequence selected from the group consisting of SEQ ID NOS:2-15,
are at least 89% identical to a nucleotide sequence selected from the group consisting of SEQ ID NOS: 2-15,
are at least 94% identical to a nucleotide sequence selected from the group consisting of SEQ ID NOS: 2-15,
comprises a nucleotide sequence selected from the group consisting of SEQ ID NOS: 2-15, or
consists of a nucleotide sequence selected from the group consisting of SEQ ID NOS: 2-15.

3. The alphavirus structural protein expression cassette of claim 1 further comprising a third recognition sequence upstream of the 3' untranslated region,
a third and a fourth recognition sequence upstream of the 3' untranslated region, or
a third, a fourth, and a fifth recognition sequence upstream of the 3' untranslated region.

4. The alphavirus structural protein expression cassette of claim 1 which encodes the alphavirus envelope polypeptide.

5. The alphavirus structural protein expression cassette of claim 1 which encodes the alphavirus capsid polypeptide.

6. The alphavirus structural protein expression cassette of claim 1 further comprising a nucleotide sequence encoding a foot-and-mouth disease virus (FMDV) 2A protease between the 5' sequence which initiates transcription of alphavirus RNA and the alphavirus subgenomic region promoter.

7. The alphavirus structural protein expression cassette of claim 1 further comprising a selectable marker located 5' to the alphavirus subgenomic region promoter.

8. The alphavirus structural protein expression cassette of claim 1 wherein at least one replicase recognition sequence is derived from an alphavirus selected from the group consisting of Sindbis virus, Venezuelan equine encephalitis virus, Semliki Forest virus, Ross River virus, and Western equine encephalitis virus.

9. The alphavirus structural protein expression cassette of claim 1 wherein the nucleotide sequence encodes the alphavirus envelope polypeptide, wherein the alphavirus is selected from the group consisting of Sindbis virus, Venezuelan equine encephalitis virus, Semliki Forest virus, and Western equine encephalitis virus.

10. The alphavirus structural protein expression cassette of claim 1 wherein the nucleotide sequence encodes the alphavirus capsid polypeptide, wherein the alphavirus is selected from the group consisting of Sindbis virus, Venezuelan equine encephalitis virus, Semliki Forest virus, Ross River virus, and Western equine encephalitis virus.

11. A packaging cell comprising a first alphavirus structural protein expression cassette of claim 1.

12. The packaging cell of claim 11 further comprising a second alphavirus structural protein expression cassette of claim 1, wherein the first alphavirus structural protein expression cassette encodes the alphavirus envelope polypeptide and the second alphavirus structural protein expression cassette encodes the alphavirus capsid polypeptide.

13. The packaging cell of claim 11 which is a PerC6 cell.

14. A method of producing a packaging cell for production of alphavirus particles, comprising:
introducing into a host cell a first alphavirus structural protein expression cassette of claim 1 which encodes the alphavirus envelope polypeptide; and
introducing into the host cell a second alphavirus structural protein expression cassette of claim 1 which encodes the alphavirus capsid polypeptide.

15. A method of producing alphavirus particles, comprising:
providing a packaging cell comprising a first and a second alphavirus structural protein expression cassette of claim 1, wherein the first alphavirus structural protein expression cassette encodes the alphavirus envelope polypeptide and the second alphavirus structural protein expression cassette encodes the alphavirus capsid polypeptide; and
introducing into the packaging cell a vector selected from the group consisting of an alphavirus vector construct, an RNA vector replicon, a eukaryotic layered vector initiation system, and an alphavirus vector particle,
whereby alphavirus particles are produced.

## Patentansprüche

1. Alphavirus-Strukturprotein-Expressionskassette, umfassend von 5' nach 3':
eine 5'-Sequenz, die die Transkription von Alphavirus-RNA initiiert;
einen Alphavirus-subgenomischer-Bereich-Promotor;
eine entweder für ein Alphavirus-Hüllpolypeptid oder ein Alphavirus-Capsidpolypeptid codierende Nukleotidsequenz;
einen 3' nicht translatierten Bereich; und
einen PolyA-Trakt,
wobei die Alphavirus-Strukturprotein-Expressionskassette eine neben dem PolyA-Trakt liegende erste Replikase-Erkennungssequenz und eine zwischen der ersten Replikase-Erkennungssequenz und der Nukleotidsequenz liegende zweite Replikase-Erkennungssequenz umfasst.

2. Alphavirus-Strukturprotein-Expressionskassette nach Anspruch 1, wobei die erste und die zweite Replikase-Erkennungssequenz zu wenigstens 78% mit einer aus der aus SEQ ID NO: 2-15 bestehenden Gruppe ausgewählten Nukleotidsequenz identisch sind,
zu wenigstens 84% mit einer aus der aus SEQ ID NO: 2-15 bestehenden Gruppe ausgewählten Nukleotidsequenz identisch sind,
zu wenigstens 89% mit einer aus der aus SEQ ID NO: 2-15 bestehenden Gruppe ausgewählten Nukleotidsequenz identisch sind,
zu wenigstens 94% mit einer aus der aus SEQ ID NO: 2-15 bestehenden Gruppe ausgewählten Nukleotidsequenz identisch sind,
eine aus der aus SEQ ID NO: 2-15 bestehenden Gruppe ausgewählte Nukleotidsequenz umfasst oder aus einer aus der aus SEQ ID NO: 2-15 bestehenden Gruppe ausgewählten Nukleotidsequenz besteht.

3. Alphavirus-Strukturprotein-Expressionskassette nach Anspruch 1, ferner umfassend eine dritte stromaufwärts des nicht translatierten 3'-Bereichs liegende Erkennungssequenz,
eine dritte und eine vierte stromaufwärts des nicht translatierten 3'-Bereichs liegende Erkennungssequenz oder
eine dritte, eine vierte und eine fünfte stromaufwärts des nicht translatierten 3'-Bereichs liegende Erkennungssequenz.

4. Alphavirus-Strukturprotein-Expressionskassette nach Anspruch 1, die für das Alphavirus-Hüllpolypeptid codiert.

5. Alphavirus-Strukturprotein-Expressionskassette nach Anspruch 1, die für das Alphavirus-Capsidpolypeptid codiert.

6. Alphavirus-Strukturprotein-Expressionskassette nach Anspruch 1, ferner umfassend eine für eine FMDV(Maul-und-Klauenseuche-Virus)-2A-Protease codierende Nukleotidsequenz zwischen der 5`-Sequenz, die die Transkription von Alphavirus-RNA initiiert, und dem Alphavirus-subgenomischer-Bereich-Promotor.

7. Alphavirus-Strukturprotein-Expressionskassette nach Anspruch 1, ferner umfassend einen 5' zu dem Alphavirus-subgenomischer-Bereich-Promotor liegenden selektionierbaren Marker.

8. Alphavirus-Strukturprotein-Expressionskassette nach Anspruch 1, wobei wenigstens eine Replikase-Erkennungssequenz aus einem aus der aus Sindbis-Virus, Venezuelan Equine Encephalitis-Virus, Semliki Forest-Virus, Ross River-Virus und Western Equine Encephalitis-Virus bestehenden Gruppe ausgewählten Alphavirus stammt.

9. Alphavirus-Strukturprotein-Expressionskassette nach Anspruch 1, wobei die Nukleotidsequenz für das Alphavirus-Hüllpolypeptid codiert, wobei das Alphavirus aus der aus Sindbis-Virus, Venezuelan Equine Encephalitis-Virus, Semliki Forest-Virus, und Western Equine Encephalitis-Virus bestehenden Gruppe ausgewählt ist.

10. Alphavirus-Strukturprotein-Expressionskassette nach Anspruch 1, wobei die Nukleotidsequenz für das Alphavirus-Capsidpolypeptid codiert, wobei das Alphavirus aus der aus Sindbis-Virus, Venezuelan Equine Encephalitis-Virus, Semliki Forest-Virus, Ross River-Virus und Western Equine Encephalitis-Virus bestehenden Gruppe ausgewählt ist.

11. Verpackungszelle, umfassend eine erste Alphavirus-Strukturprotein-Expressionskassette nach Anspruch 1.

12. Verpackungszelle nach Anspruch 11, ferner umfassend eine zweite Alphavirus-Strukturprotein-Expressionskassette nach Anspruch 1, wobei die erste Alphavirus-Strukturprotein-Expressionskassette für das Alphavirus-Hüllpolypeptid und die zweite Alphavirus-Strukturprotein-Expressionskassette für das Alphavirus-Capsidpolypeptid codiert.

13. Verpackungszelle nach Anspruch 11, bei der es sich um eine PerC6-Zelle handelt.

14. Verfahren zur Herstellung einer Verpackungszelle für die Produktion von Alphaviruspartikeln, bei dem man:
in eine Wirtszelle eine erste Alphavirus-Strukturprotein-Expressionskassette nach Anspruch 1, die für das Alphavirus-Hüllpolypeptid codiert, einführt; und
in die Wirtszelle eine zweite Alphavirus-Strukturprotein-Expressionskassette nach Anspruch 1, die für das Alphavirus-Capsidpolypeptid codiert, einführt.

15. Verfahren zur Produktion von Alphaviruspartikeln, bei dem man:
eine eine erste und eine zweite Alphavirus-Strukturprotein-Expressionskassette nach Anspruch 1 umfassende Verpackungszelle bereitstellt, wobei die erste Alphavirus-Strukturprotein-Expressionskassette für das Alphavirus-Hüllpolypeptid und die zweite Alphavirus-Strukturprotein-Expressionskassette für das Alphavirus-Capsidpolypeptid codiert; und
in die Verpackungszelle einen aus der aus einem Alphavirus-Vektorkonstrukt, einem RNA-Vektor-Replikon, einem eukaryontischen geschichteten Vektorinitiationssystem und einem Alphavirus-Vektorpartikel bestehenden Gruppe ausgewählten Vektor einführt,
wodurch Alphaviruspartikel produziert werden.

## Revendications

1. Cassette d'expression d'une protéine structurale d'alphavirus comprenant, de 5' vers 3' :
une séquence en 5' qui débute la transcription d'un ARN d'alphavirus ;
un promoteur d'une région sous-génomique d'alphavirus ;
une séquence nucléotidique codant soit pour un polypeptide de l'enveloppe d'un alphavirus soit pour un polypeptide de la capside d'un alphavirus ;
une région non traduite en 3' ; et
un système de polyA,
dans laquelle la cassette d'expression d'une protéine structurale d'alphavirus comprend une première séquence de reconnaissance d'une réplicase, qui est adjacente au système de polyA, ainsi qu'une deuxième séquence de reconnaissance d'une réplicase, qui est située entre la première séquence de reconnaissance d'une réplicase et la séquence nucléotidique.

2. Cassette d'expression d'une protéine structurale d'alphavirus selon la revendication 1, dans laquelle les première et deuxième séquences de reconnaissance d'une réplicase sont identiques, au moins à 78%, à une séquence nucléotidique choisie dans le groupe constitué par les SEQ ID n° : 2 à 15,
sont identiques, au moins à 84%, à une séquence nucléotidique choisie dans le groupe constitué par les SEQ ID n° : 2 à 15,
sont identiques, au moins à 89%, à une séquence nucléotidique choisie dans le groupe constitué par les SEQ ID n° : 2 à 15,
sont identiques, au moins à 94%, à une séquence nucléotidique choisie dans le groupe constitué par les SEQ ID n° : 2 à 15,
comprennent une séquence nucléotidique choisie dans le groupe constitué par les SEQ ID n° : 2 à 15 ou
sont constituées par une séquence nucléotidique choisie dans le groupe constitué par les SEQ ID n° : 2 à 15.

3. Cassette d'expression d'une protéine structurale d'alphavirus, selon la revendication 1, comprenant en outre une troisième séquence de reconnaissance en amont de la région non traduite en 3'
une troisième et une quatrième séquences de reconnaissance en amont de la région non traduite en 3' ou
une troisième, une quatrième et une cinquième séquences de reconnaissance en amont de la région non traduite en 3'.

4. Cassette d'expression d'une protéine structurale d'alphavirus, selon la revendication 1, qui code pour le polypeptide de l'enveloppe d'un alphavirus.

5. Cassette d'expression d'une protéine structurale d'alphavirus, selon la revendication 1, qui code pour le polypeptide de la capside d'un alphavirus.

6. Cassette d'expression d'une protéine structurale d'alphavirus, selon la revendication 1, comprenant en outre une séquence nucléotidique qui code pour une protéase 2A du virus de la fièvre aphteuse (FMDV) entre la séquence située en 5', qui débute la transcription de l'ARN d'un alphavirus, et le promoteur d'une région sous-génomique d'alphavirus.

7. Cassette d'expression d'une protéine structurale d'alphavirus, selon la revendication 1, comprenant en outre un marqueur sélectionnable situé en 5' du promoteur d'une région sous-génomique d'alphavirus.

8. Cassette d'expression d'une protéine structurale d'alphavirus selon la revendication 1, dans laquelle au moins une séquence de reconnaissance d'une réplicase est dérivée d'un alphavirus choisi dans le groupe constitué par les virus Sindbis, virus de l'encéphalite équine du Venezuela, virus de la forêt de Semliki, virus de la rivière Ross et virus de l'encéphalite équine de l'Ouest.

9. Cassette d'expression d'une protéine structurale d'alphavirus selon la revendication 1, dans laquelle la séquence nucléotidique code pour le polypeptide de l'enveloppe d'un alphavirus, dans laquelle l'alphavirus est choisi dans le groupe constitué par les virus Sindbis, virus de l'encéphalite équine du Venezuela, virus de la forêt de Semliki et virus de l'encéphalite équine de l'Ouest.

10. Cassette d'expression d'une protéine structurale d'alphavirus selon la revendication 1, dans laquelle la séquence nucléotidique code pour le polypeptide de la capside d'un alphavirus, dans laquelle l'alphavirus est choisi dans le groupe constitué par les virus Sindbis, virus de l'encéphalite équine du Venezuela, virus de la forêt de Semliki, virus de la rivière Ross et virus de l'encéphalite équine de l'Ouest.

11. Cellule d'encapsidation comprenant une première cassette d'expression d'une protéine structurale d'alphavirus selon la revendication 1.

12. Cellule d'encapsidation, selon la revendication 11, comprenant en outre une deuxième cassette d'expression d'une protéine structurale d'alphavirus, selon la revendication 1, dans laquelle la première cassette d'expression d'une protéine structurale d'alphavirus code pour le polypeptide de l'enveloppe d'un alphavirus et la deuxième cassette d'expression d'une protéine structurale d'alphavirus code pour le polypeptide de la capside d'un alphavirus.

13. Cellule d'encapsidation, selon la revendication 11, qui est une cellule PerC6.

14. Procédé de production d'une cellule d'encapsidation destinée à produire des particules d'alphavirus, comprenant les étapes consistant à :
introduire dans une cellule hôte une première cassette d'expression d'une protéine structurale d'alphavirus, selon la revendication 1, qui code pour le polypeptide de l'enveloppe d'un alphavirus ; et
introduire dans la cellule hôte une deuxième cassette d'expression d'une protéine structurale d'alphavirus, selon la revendication 1, qui code pour le polypeptide de la capside d'un alphavirus.

15. Procédé de production de particules d'alphavirus, comprenant les étapes consistant à :
fournir une cellule d'encapsidation comprenant une première et une deuxième cassettes d'expression d'une protéine structurale d'alphavirus, selon la revendication 1, dans laquelle la première cassette d'expression d'une protéine structurale d'alphavirus code pour le polypeptide de l'enveloppe d'un alphavirus et la deuxième cassette d'expression d'une protéine structurale d'alphavirus code pour le polypeptide de la capside d'un alphavirus ; et
introduire dans la cellule d'encapsidation un vecteur choisi dans le groupe constitué par un construit de vecteur d'alphavirus, un réplicon de vecteur à ARN, un système d'initiation d'un vecteur multicouches eucaryote et une particule de vecteur d'alphavirus,
moyennant quoi des particules d'alphavirus sont produites.
